# EUROPEAN PATENT SPECIFICATION

(11) **EP 3 532 081 B1**
(45) Date of publication and mention of the grant of the patent: **30.08.2023**
(21) Application number: 17804944.1
(22) Date of filing: 30.10.2017
(51) Int. Cl.: A61K 35/74, A61P 1/16, A61P 3/06

(54) **COMPOSITION FOR THE PREVENTIVE OR CURATIVE TREATMENT OF LIVER DISORDERS**
ZUSAMMENSETZUNG ZUR PRÄVENTIVEN ODER KURATIVEN BEHANDLUNG VON ERKRANKUNGEN DER LEBER
COMPOSITION POUR LE TRAITEMENT PRÉVENTIF OU CURATIF DE TROUBLES HÉPATIQUES

(30) Priority: 28.10.2016 IT 201600109507
(43) Date of publication of application: 04.09.2019
(73) Proprietor: Probiotical S.P.A., 28100 Novara NO (IT)
(72) Inventor: MOGNA, Giovanni, 28100 Novara (NO) (IT)
(74) Representative: Benedetto, Marco
(86) International application number: PCT/IB2017/056728
(87) International publication number: WO 2018/078599

(56) References cited:
- WO-A1-2015/172191
- DATABASE WPI Week 201265 Thomson Scientific, London, GB; AN 2012-M21299 XP002770494, & JP 2012 180373 A (MEGMILK SNOW BRAND CO LTD) 20 September 2012 (2012-09-20)
- DATABASE WPI Week 201559 Thomson Scientific, London, GB; AN 2015-380973 XP002770495, & KR 2015 0068670 A (KOREA YAKULT CO LTD) 22 June 2015 (2015-06-22)
- MCMILLIN B M ET AL: "RELIABLE DISTRIBUTED SORTING THROUGH THE APPLICATION-ORIENTED FAULT TOLERANCE PARADIGM", IEEE TRANSACTIONS ON PARALLEL AND DISTRIBUTED SYSTEMS, IEEE SERVICE CENTER, LOS ALAMITOS, CA, US, vol. 3, no. 4, 1 July 1992 (1992-07-01), pages 411-420, XP000293564, ISSN: 1045-9219, DOI: 10.1109/71.149960
- JINJIN CHEN ET AL: "Bifidobacterium adolescentis supplementation ameliorates visceral fat accumulation and insulin sensitivity in an experimental model of the metabolic syndrome", BRITISH JOURNAL OF NUTRITION, vol. 107, no. 10, 14 September 2011 (2011-09-14), pages 1429-1434, XP055179451, ISSN: 0007-1145, DOI: 10.1017/S0007114511004491

## Description

The present invention relates to a composition (C) comprising probiotic bacteria of the genus *Bifidobacterium* for the curative or preventive treatment of liver disorders, particularly in subjects of paediatric age, and to a pharmaceutical preparation or medical device comprising said composition. Paediatric non-alcoholic fatty liver disease (NAFLD) may progress from *non-alcoholic* fatty liver (NAFL) to *non-alcoholic steatohepatitis* (NASH).

Non-alcoholic fatty liver disease (NAFLD) is the most frequent cause of chronic liver disease in adults and children. It is considered to be a set of liver conditions ranging from fatty liver to *non-alcoholic steatohepatitis* (NASH) with or without fibrosis. The pathogenesis of NAFLD has been partially clarified and it is generally believed that, from a pathological standpoint, it is a multi-factor disease. This assumption is based on the central role of the high circulating levels of free fatty acids (FFAs) and insulin resistance, which can interact with each other, causing an accumulation of FFAs in hepatocytes (*non-alcoholic* fatty liver, NAFL), oxidative stress and inflammatory response, thus providing the basis for the progression of liver damage.

It is interesting to note that various studies shown that intestinal microbiota may also play an important role in the development and progression of NAFLD. In the context of the present invention, "microbiota" means the ecological community of commensal, symbiotic and pathogenic microorganisms that literally share the space of our body and the internal cavities thereof. Human microbiota consists of about 100 trillion microbial cells which exceed human cells in a ratio of 100 to 1, with five phyla representing the majority of the community. About 160 species have been found in the large intestine of each individual and very few of them are shared by individuals who are not related. It has been demonstrated that the microbiota composition can influence the proportion of calories absorbed by intestinal contents.

In fact, bacteria play an important role in the absorption and emulsion of fats and liposoluble vitamins in the small intestine. Intestinal microbiota can also produce endotoxins, such as lipopolysaccharide (LPS), which, in a condition of compromised intestinal permeability, can reach the circulatory system, leading to an intra- and extrahepatic inflammatory response, which in turn induces a progression from fatty liver to NASH.

Intestinal microbiota is influenced by diet, age, infections, hygienic habits and antibiotic therapy. Patients affected by NAFLD have shown a high prevalence of small intestine bacterial overgrowth (SIBO) and a greater intestinal permeability, which favours bacterial translocation, exposing the liver to toxic microbial metabolites. Obese patients affected by NAFLD have shown a reduction in *Bacteroidetes* and an increase in *Firmicutes* compared to patients of normal weight.

Surprisingly, the weight loss due to a limited intake of carbohydrates and fats increases the prevalence of *Bacteroidetes* in the intestinal microbiota.

Furthermore, studies on mice affected by NAFLD have demonstrated that some probiotic bacteria reduce the concentration of cholesterol in plasma and protect against the accumulation of fats in adipose tissue and hepatocytes.

Although recent studies have highlighted that intestinal microbiota have a role in the pathogenesis of NAFLD, how such results may improve therapy remains to be clarified.

An understanding of the nature of intestinal dysbiosis, the integrity of the intestinal barrier and the mechanisms of the hepatic immune response to intestine-derived factors is potentially relevant for the development of new therapies for the treatment of chronic liver diseases.

The term "dysbiosis" was coined by Metchnikoff in 1907 to describe altered pathogenic bacteria in the intestine. Dysbiosis has been defined as indicative of the qualitative and quantitative changes in the intestinal flora and in the metabolic and local distribution thereof.

In a recent in-depth characterisation of the intestinal microbiotic profile of children affected by NAFLD versus obese and thin children without liver disease it emerged that *Ruminococcus* and *Dorea* increase in concomitance with the progression of NAFL towards NASH.

However, the therapies presently available for the treatment and/or prevention of NAFLD in order to avoid its progression to NASH and for the curative treatment of NASH are based on radical modifications of the diet, the administration of drugs that act on glycaemia (metformin and thiazolidinediones) or statins, or surgical weight reduction (bariatric surgery).

WO 2015/172191 A1 discloses methods for the treatment of metabolic syndrome and/or insulin resistance, and for the treatment or prevention of obesity an obesity-related disorders, comprising the administration of two or more probiotic bacterial strains selected from the Lactobacillus genus and the Bifidobacterium genus.

JP 2012 180373 A reports on the potential use of *Bifidobacterium longum* for treating metabolic disorders associated with aging.

KR 2015 0068670 A describes the possible antidiabetic use of *Bifidobacterium longum strain* in combination with *Lactobacillus gasseri strain.*

There is thus a felt need to provide a treatment for NAFLD and/or NASH, above all in subjects of paediatric age (0-18 years), which has a low impact on lifestyle, does not require surgical intervention and is practically free of the side effects normally associated with the above-mentioned pharmacological treatments.

In response to this need, the present invention provides a composition (C) which comprises an effective amount of a mixture comprising, or consisting of, at least one bacterial strain for use in the preventive and/or curative treatment of symptoms and/or pathologies deriving from, or connected to metabolic syndrome, enlarged liver, non-alcoholic fatty liver disease (NAFLD) and/or non-alcoholic steatohepatitis (NASH) in a subject, wherein said treatment comprises oral administration of said composition (C) to the subject, wherein said at least one bacterial strain is selected from among the bacterial strains belonging to the species B. adolescentis selected from the group comprising, or consisting of:
- (i) Bifidobacterium adolescentis BA06, deposited with the Institute DSMZ in Germany by Probiotical S.p.A. on 7.4.2017 and having the deposit no. 32479; and
- (ii) Bifidobacterium adolescentis BA07, deposited with the Institute DSMZ in Germany by Probiotical S.p.A. on 21.4.2017 and having the deposit no. 32491; or from among the bacterial strains belonging to the species B. bifidum selected from the group comprising, or consisting of:
- (iii) Bifidobacterium bifidum BB07, deposited with the Institute DSMZ in Germany by Probiotical S.p.A. on 7.4.2017 and having the deposit no. 32480; and
- (iv) Bifidobacterium bifidum BB08, deposited with the Institute DSMZ in Germany by Probiotical S.p.A. on 7.4.2017 and having the deposit no. 32481; or from among the bacterial strains belonging to the species B. longum selected from the group comprising, or consisting of:
- (v) Bifidobacterium longum BL22, deposited with the Institute DSMZ in Germany by Probiotical S.p.A. on 7.4.2017 and having the deposit no. 32482; and
- (vi) Bifidobacterium longum BL23, deposited with the Institute DSMZ in Germany by Probiotical S.p.A. on 7.4.2017 and having the deposit no. 32483.

The present invention responds to said need, moreover, by means of a pharmaceutical preparation or medical device or food supplement comprising a composition (C) which comprises (I) a mixture comprising, or consisting of, at least one bacterial strain selected from among the bacterial strains belonging to the species B. adolescentis selected from the group comprising, or consisting of:
- (i) Bifidobacterium adolescentis BA06, deposited with the Institute DSMZ in Germany by Probiotical S.p.A. on 7.4.2017 and having the deposit no. 32479; and
- (ii) Bifidobacterium adolescentis BA07, deposited with the Institute DSMZ in Germany by Probiotical S.p.A. on 21.4.2017 and having the deposit no. 32491; or from among the bacterial strains belonging to the species B. bifidum selected from the group comprising, or consisting of:
- (iii) Bifidobacterium bifidum BB07, deposited with the Institute DSMZ in Germany by Probiotical S.p.A. on 7.4.2017 and having the deposit no. 32480; and
- (iv) Bifidobacterium bifidum BB08, deposited with the Institute DSMZ in Germany by Probiotical S.p.A. on 7.4.2017 and having the deposit no. 32481; or from among the bacterial strains belonging to the species B. longum selected from the group comprising, or consisting of:
- (v) Bifidobacterium longum BL22, deposited with the Institute DSMZ in Germany by Probiotical S.p.A. on 7.4.2017 and having the deposit no. 32482; and
- (vi) Bifidobacterium longum BL23, deposited with the Institute DSMZ in Germany by Probiotical S.p.A. on 7.4.2017 and having the deposit no. 32483; and (II) at least one excipient suitable for pharmaceutical and/or food use.

It has surprisingly been found that strains of probiotic bacteria of the present invention are effective as a treatment for subjects affected by NAFLD and/or NASH, above all subjects of paediatric age.

The present invention relates to bacterial strains belonging to the species *B*. *adolescentis* selected from the group comprising or, alternatively, consisting of: (i) *Bifidobacterium adolescentis* BA06, deposited with the Institute DSMZ in Germany by Probiotical S.p.A. on 7.4.2017 and having the deposit no. 32479; and (ii) *Bifidobacterium adolescentis* BA07, deposited with the Institute DSMZ in Germany by Probiotical S.p.A. on 21.4.2017 and having the deposit no. 32491.

The present invention relates to bacterial strains belonging to the species B. *bifidum* selected from the group comprising or, alternatively, consisting of: (iii) *Bifidobacterium bifidum* BB07, deposited with the Institute DSMZ in Germany by Probiotical S.p.A. on 7.4.2017 and having the deposit no. 32480; and (iv) *Bifidobacterium bifidum* BB08, deposited with the Institute DSMZ in Germany by Probiotical S.p.A. on 7.4.2017 and having the deposit no. 32481.

The present invention relates to bacterial strains belonging to the species B. *longum* selected from the group comprising or, alternatively, consisting of: (v) *Bifidobacterium longum* BL22, deposited with the Institute DSMZ in Germany by Probiotical S.p.A. on 7.4.2017 and having the deposit no. 32482; and (vi) *Bifidobacterium longum* BL23, deposited with the Institute DSMZ in Germany by Probiotical S.p.A. on 7.4.2017 and having the deposit no. 32483.

Advantageously the bacterial strains belonging to the above-mentioned species *Bifidobacterium adolescentis*, *Bifidobacterium bifidum* and *Bifidobacterium longum,* indicated as (i) to (vi), are for use in the preventive and/or curative treatment of symptoms and/or pathologies deriving from, or connected to metabolic syndrome and enlarged liver, non-alcoholic fatty liver disease (NAFLD) and/or non-alcoholic steatohepatitis (NASH) in a subject; said treatment preferably comprises oral administration of said strains to the subject.

The composition for use according to the present invention may further be used advantageously in the preventive and/or curative treatment of symptoms and/or pathologies deriving from, or connected to metabolic syndrome and enlarged liver.

In one embodiment, the composition C1 of the present invention comprises a mixture of bacteria which comprises or, alternatively, consists of the bacterial strains (i) + (iii) + (v) and, optionally, suitable pharmaceutical and/or food grade excipients, preferably in a ratio by weight of 1:1:1, for use in the preventive and/or curative treatment of symptoms and/or pathologies deriving from, or connected to metabolic syndrome and enlarged liver, non-alcoholic fatty liver disease (NAFLD) and/or non-alcoholic steatohepatitis (NASH) in a subject; said treatment preferably comprises oral administration of said composition to the subject.

In another embodiment, the composition C2 of the present invention comprises a mixture of bacteria which comprises or, alternatively, consists of the bacterial strains (ii) + (iv) + (vi) and, optionally, suitable pharmaceutical and/or food grade excipients, preferably in a ratio by weight of 1:1:1, for use in the preventive and/or curative treatment of symptoms and/or pathologies deriving from, or connected to metabolic syndrome and enlarged liver, non-alcoholic fatty liver disease (NAFLD) and/or non-alcoholic steatohepatitis (NASH) in a subject; said treatment preferably comprises oral administration of said composition to the subject.

In another embodiment, the composition C3 of the present invention comprises a mixture of bacteria which comprises or, alternatively, consists of the bacterial strains [(i)+(ii)] + [(iii)+(iv)] + [(v)+(vi)], and, optionally, suitable pharmaceutical and/or food grade excipients, preferably in a ratio by weight of 1:1:1 ([(i)+(ii)] : [(iii)+(iv)] : [(v)+(vi)]), for use in the preventive and/or curative treatment of symptoms and/or pathologies deriving from, or connected to metabolic syndrome and enlarged liver, non-alcoholic fatty liver disease (NAFLD) and/or non-alcoholic steatohepatitis (NASH) in a subject; said treatment preferably comprises oral administration of said composition to the subject.

In the context of the present invention, metabolic syndrome (also called syndrome X, insulin resistance syndrome, CHAOS, in Australia, or Reaven's syndrome) means a clinical situation of high cardiovascular risk which comprises a series of risk factors and symptoms that manifest themselves simultaneously in an individual. These are often correlated to the person's lifestyle (excessive weight, sedentary life) or preexisting pathological situations (obesity, hypercholesterolaemia - presence of a high blood cholesterol level, etc.).

The definition most widely known and applied in clinical practice is that of the National Cholesterol Education Program Adult Treatment Panel (ATP) III of 2001. It does not consider any direct or indirect diagnostic element of insulin resistance but envisages the simultaneous presence of three variables among the following: abdominal obesity, hypertension, hypertriglyceridaemia, low HDL cholesterol and glycaemia >110 mg/dl (also including diabetes).

In the context of the present invention, "enlarged liver", or hepatomegaly, means an increase in the size of the liver compared to what is judged clinically normal for an individual; it may be detected, for example, by palpation, exams with techniques such as ultrasound or computed tomography (CT) and/or blood tests. The compositions for use according to the present invention, such as, for example, compositions C1, C2 and C3 can be advantageously used, moreover, in obese or overweight subjects, according to the definition of the World Health Organisation, i.e. with a body mass equal to or greater than 25 to 29.99 (overweight) or equal to or greater than 30 (obese), preferably in paediatric age.

In one aspect of the invention, the composition (C), such as, for example, compositions C1, C2 and C3, are administered to subjects at the risk of developing NAFLD and/or NASH, even if they do not yet have all the symptoms of said pathologies.

Preferably, the composition (C), such as, for example, compositions C1, C2 and C3 for use according to the invention, may further comprise bacterial strains belonging to the genus *Oscillospira.*

In one aspect, the present invention provides a pharmaceutical preparation or medical device or food supplement comprising a composition (C) which comprises (i) a mixture of at least one bacterial strain belonging to at least one species selected from among the group comprising, or, alternatively, consisting of, bacteria belonging to at least one species among *Bifidobacterium longum, Bifidobacterium adolescentis* and *Bifidobacterium bifidum, as defined above* and (ii) at least one excipient suitable for pharmaceutical and/or food use.

Preferably, in the pharmaceutical preparation or medical device or food supplement according to the present invention, the mixture (i) of the composition (C), such as, for example, of compositions C1, C2 and C3, comprises a mixture of bacterial strains as selected from the strains defined above, and (ii) at least one excipient suitable for pharmaceutical and/or food use, i.e. where bacteria belonging to at least each of said species are present.

Preferably, in the pharmaceutical preparation or medical device or food supplement according to the present invention, the mixture (i) of the composition (C), such as, for example, of compositions C1, C2 and C3, further comprises bacteria belonging to the genus *Oscillospira.*

The at least one excipient (ii) suitable for pharmaceutical and/or food use may be selected from among all the substances known to the person skilled in the pharmaceutical field or the field of food preparations, including, for examples, preservatives, thickeners, sweeteners, colourants, natural and artificial flavouring, antioxidants, stabilisers, fillers and mixtures thereof.

In the context of the present invention, the pharmaceutical preparation or medical device or food supplement may further comprise at least one pharmaceutically active ingredient or bacteria belonging to a species other than *Bifidobacterium longum*, *Bifidobacterium adolescentis* and *Bifidobacterium bifidum.* The following examples are provided to illustrate practical aspects of implementation.

Bifidobacteria and lactobacilli were analysed by pyrosequencing of 16S rRNA in stool samples taken from 61 obese children with NAFL and NASH and 54 healthy controls.

Three species of Bifidobacterium (*Bifidobacterium longum*, *Bifidobacterium bifidum*, *Bifidobacterium adolescentis*) and five species of Lactobacillus (*Lactobacillus zeae*, *Lactobacillus vaginalis*, *Lactobacillus brevis*, *Lactobacillus ruminis*, *Lactobacillus mucosae*) were frequently found in the metagenomic analyses. The species of *Lactobacillus* were in greater number in the subjects affected by NAFL, with or without NASH, than in the controls, whilst bifidobacteria were frequent in healthy subjects.

### MATERIALS AND METHODS

The study was conducted on 61 consecutive overweight or obese children (35 males, age 11.45 ± 2.42 years) monitored at the Department of Hepato-Metabolic Diseases (Rome, Italy) of the Bambino Gesù Paediatric Hospital and Research Institute (OPBG) in 2013. All patients underwent testing for secondary causes of fatty liver, such as hepatitis, cytomegalovirus and Epstein-Barr viral infections, liver disease, autoimmune diseases, metabolic liver disease, Wilson's disease, coeliac disease and alpha-1-antitrypsin deficiency, which were ruled out using standard histological, laboratory and clinical criteria. Fifty-three patients, with a BMI>95th percentile, and/or *hypertransaminasaemia* and steatosis, underwent a liver biopsy for the assessment of liver damage. The study population was made up of 3 groups: 8 (13.12%) obese, 27 (44.26%) with NAFLD and 26 (42.62%) with defined NASH. Furthermore, 54 healthy children of a corresponding age, enrolled at the Human Microbiome Unit of the OPBG, were used as controls (control subjects).

The hospital's research ethics committee approved the study, which was conducted in conformity with the Declaration of Helsinki (according to the revision of Seoul, Korea, October 2008). The parents of the enrolled subjects gave informed written consent. The study was approved by the ethics committee of the OPBG (reference no. 768.12).

Stool samples were collected from all of the affected children and control subjects and analysed at the Human Microbiome Unit of the OPBG.

### Anthropometric and laboratory analyses

Anthropometric parameters including weight, height, BMI and waist circumference were measured in all of the children using standardised methods. Obesity was defined as a BMI ≥ 95th percentile, adjusted for age and sex according to WHO growth graphs. Fasting plasma glucose (FPG), alanine aminotransferase (ALT), aspartate aminotransferase (AST), γ-glutamyltransferase (GGT), fasting serum triglycerides (TG) and total cholesterol levels were determined using standardised methods in our laboratory. Insulin was measured by means of radioimmunoassay (MYRIA Technogenetics, Milan, Italy).

The degree of insulin resistance/sensitivity was determined by evaluating the homeostatic model (HOMA). A HOMA value > 2 was considered indicative of insulin resistance.

All of the patients underwent abdominal ultrasound examination.

Designated expert radiologists, unaware of the clinical conditions of the patients, performed a liver ultrasound scan after overnight fasting.

The liver was analysed in terms of fatty liver markers using established criteria: a bright liver echostructure (compared with the echo response of the right kidney).

### DNA extraction

Each stool sample collected from the 115 children underwent a further metagenomic analysis having 16S-rRNA as the target. DNA was extracted from each sample using the QIAamp DNA Stool Mini Kit (Qiagen, Germany).

The stools were resuspended in 1.5 ml PBS, rendered homogeneous by mixing for 2 minutes and centrifuged at 20,800×g. After removal of the supernatant, the sediment was resuspended in 500 µl of PBS, to which 500 µl of beads/PBS were added (1 mg/µl, w/v) (acid-washed glass beads, SigmaAldrich). The supernatant was collected and treated for one freeze/thaw cycle (-20 C/70°C) for 20 minutes in each phase as previously described. After centrifugation at 5,200×g for 5 minutes, the supernatant was subjected to extraction with the QlAamp DNA Stool Mini Kit (Qiagen, Germany) according to the manufacturer's instructions and subsequently subjected to a metagenomic analysis pipeline. The intestinal microbiome was examined by pyrosequencing the V1-V3 regions of the 16S rRNA gene (amplicon size 520 bp), on a GS Junior platform (454 Life Sciences, Roche Diagnostics, Italy).

### Results

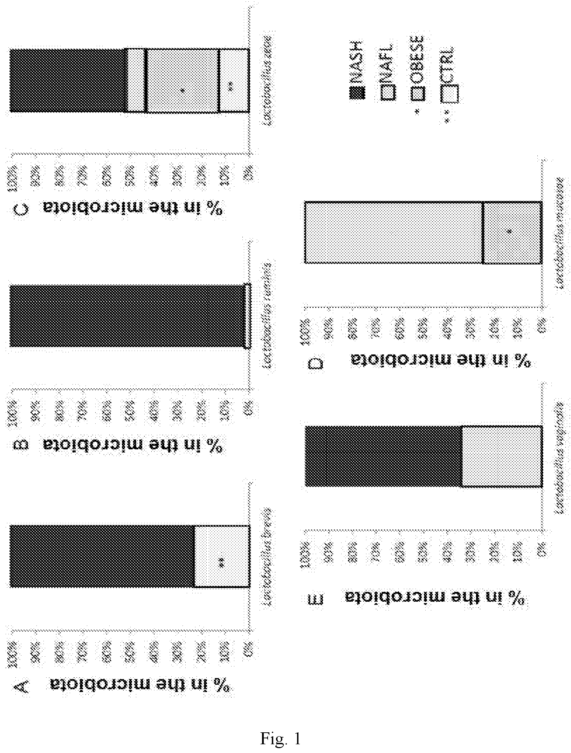

The analysis revealed a prevalent distribution of 3 main species of *Bifidobacterium* (*Bifidobacterium longum*, *Bifidobacterium bifidum*, *Bifidobacterium adolescentis*) and 5 species of Lactobacillus *(Lactobacillus zeae, Lactobacillus vaginalis, Lactobacillus brevis, Lactobacillus ruminis, Lactobacillus mucosae*), which appeared in a different manner in the targeted metagenomic analyses. In particular, *L*. *zeae* was present in all four groups (i.e. obese, control -CTRL-, NAFL and NASH children), whereas *L*. *ruminis* and *L. vaginalis* were present only in the NAFL and NASH groups. *L. mucosae* was associated with obesity and NAFL, and *L. brevis* with NASH and control subjects (Figure 1).

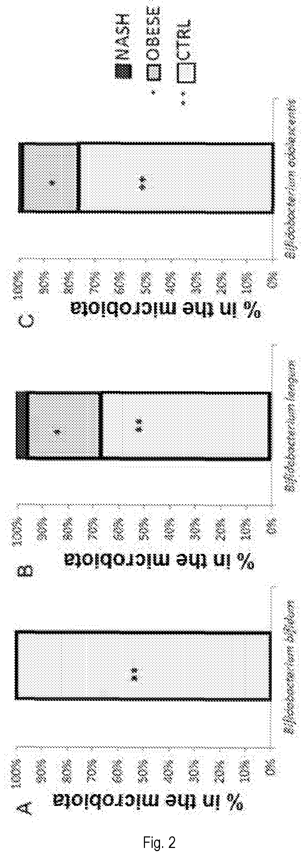

In contrast, an inverse correlation was observed between bifidobacteria and NAFL and NASH; in particular, although the species B. *longum*, B. *adolescentis* and *B. bifidum* were largely reduced in all of the patients, they showed to be present to a much larger degree in the control subjects. In fact, the species *B*. *bifidum* was present only in the group of control subjects, whereas the species *B*. *adolescentis* and *B*. longum were present to a larger degree in the control subjects and at a lower concentration in the obese and NASH groups (Figure 2).

An increase in the species of *Lactobacillus* in patients with NASH and NAFL compared to the control subjects was demonstrated in this study. These data are in disagreement with a previous experimental study demonstrating that the administration of a probiotic based on *Lactobacillus johnsonii* to rats fed a high-fat diet (HFD) was capable of preventing the development of NAFLD (Ren T, Huang C, Cheng M. "Dietary blueberry and bifidobacteria attenuate nonalcoholic fatty liver disease in rats" by affecting SIRT1-mediated signaling pathway. Oxid Med Cell Longev. 2014;2014:469059).

The discrepancy between these results and the present study may be attributed to a specific activity of lactobacilli or the use of animal models that may not fully reflect human beings. It is interesting to note that all of the obese patients enrolled in the present study showed a significant reduction in the presence of bifidobacteria compared to the control subjects, thus suggesting a possible role of bifidobacteria in this disease as well. Their role in obesity is in fact a matter of controversy, with some studies demonstrating an involvement thereof in increases in body weight and others showing opposite results. In particular, the strains of the species B. *longum* showed to be particularly capable of reducing increases in weight and body fat in a group of rats fed a high-fat diet for 5 weeks.

The species of *Lactobacillus* showed to be associated with both normal weight and obesity.

In conclusion, it was demonstrated that the strains of probiotic bifidobacteria, in particular the strains of probiotic bacteria belonging to the species *Bifidobacterium adolescentis*, *Bifidobacterium bifidum* and *Bifidobacterium longum* and lactobacilli may influence NAFLD in a different way (Table 1).

**Table 1**

| Bifidobacteria and lactobacilli: influence on NAFLD | |
|---|---|
| | Positive (X) or negative (O) influence |
| *Lactobacillus brevis* | O |
| *Lactobacillus ruminis* | O |
| *Lactobacillus zeae* | O |
| *Lactobacillus mucosae* | O |
| *Lactobacillus vaginalis* | O |
| *Bifidobacterium bifidum* | X |
| *Bifidobacterium longum* | X |
| *Bifidobacterium adolescentis* | X |

Based on the results of this study, which ascertained the absence of certain species of *Bifidbacterium* in subjects affected by, or at a risk of developing NASH, NAFL and obesity and the presence of the same species in healthy not-at-risk subjects, it was surprisingly found that several species of *Bifidobacterium*, namely, the species *B*. *adolescentis*, *B. longum* and *B*. *bifidum*, may have a role in protecting against the development of NASH, NAFL and obesity, in view of their significant reduction in patients affected by such disorders.

The administration of specific suitably selected strains of *B*. *adolescentis*, *B*. *longum* and *B*. *bifidum*, individually or in combination with *Oscillospira*, may improve the symptoms and clinical course of NAFLD, especially in obese subjects of paediatric age (0-18, preferably 1-14 years); said specific bacterial strains are preferably the ones indicated above as (i) to (vi) and the compositions of interest of the present invention are the ones indicated above as C, C1, C2, C3 and C4.

## Claims

1. Bacterial strains belonging to the species B. adolescentis selected from the group comprising, or consisting of:
- (i) Bifidobacterium adolescentis BA06, deposited with the Institute DSMZ in Germany by Probiotical S.p.A. on 7.4.2017 and having the deposit no. 32479; and
- (ii) Bifidobacterium adolescentis BA07, deposited with the Institute DSMZ in Germany by Probiotical S.p.A. on 21.4.2017 and having the deposit no. 32491.

2. Bacterial strains belonging to the species B. bifidum selected from the group comprising, or consisting of:
- (iii) Bifidobacterium bifidum BB07, deposited with the Institute DSMZ in Germany by Probiotical S.p.A. on 7.4.2017 and having the deposit no. 32480; and
- (iv) Bifidobacterium bifidum BB08, deposited with the Institute DSMZ in Germany by Probiotical S.p.A. on 7.4.2017 and having the deposit no. 32481.

3. Bacterial strains belonging to the species B. longum selected from the group comprising, or consisting of:
- (v) Bifidobacterium longum BL22, deposited with the Institute DSMZ in Germany by Probiotical S.p.A. on 7.4.2017 and having the deposit no. 32482; and
- (vi) Bifidobacterium longum BL23, deposited with the Institute DSMZ in Germany by Probiotical S.p.A. on 7.4.2017 and having the deposit no. 32483.

4. The bacterial strains according to any one of claims 1-3, wherein said bacterial strains belonging to the species Bifidobacterium adolescentis, Bifidobacterium bifidum and Bifidobacterium longum are for use in the preventive and/or curative treatment of symptoms and/or pathologies deriving from, or connected to, metabolic syndrome, enlarged liver, non-alcoholic fatty liver disease (NAFLD) and/or non-alcoholic steatohepatitis (NASH) in a subject; said treatment preferably comprises oral administration of said strains to the subject.

5. A composition (C) which comprises an effective amount of a mixture comprising, or consisting of, at least one bacterial strain selected from among the bacterial strains belonging to the species B. adolescentis selected from the group comprising, or consisting of:
- (i) Bifidobacterium adolescentis BA06, deposited with the Institute DSMZ in Germany by Probiotical S.p.A. on 7.4.2017 and having the deposit no. 32479; and
- (ii) Bifidobacterium adolescentis BA07, deposited with the Institute DSMZ in Germany by Probiotical S.p.A. on 21.4.2017 and having the deposit no. 32491; or
from among the bacterial strains belonging to the species B. bifidum selected from the group comprising, or consisting of:
- (iii) Bifidobacterium bifidum BB07, deposited with the Institute DSMZ in Germany by Probiotical S.p.A. on 7.4.2017 and having the deposit no. 32480; and
- (iv) Bifidobacterium bifidum BB08, deposited with the Institute DSMZ in Germany by Probiotical S.p.A. on 7.4.2017 and having the deposit no. 32481; or
from among the bacterial strains belonging to the species B. longum selected from the group comprising, or consisting of:
- (v) Bifidobacterium longum BL22, deposited with the Institute DSMZ in Germany by Probiotical S.p.A. on 7.4.2017 and having the deposit no. 32482; and
(vi) Bifidobacterium longum BL23, deposited with the Institute DSMZ in Germany by Probiotical S.p.A. on 7.4.2017 and having the deposit no. 32483;
for use in the preventive and/or curative treatment of symptoms and/or pathologies deriving from, or connected to, metabolic syndrome, enlarged liver, non-alcoholic fatty liver disease (NAFLD) and/or non-alcoholic steatohepatitis (NASH) in a subject, wherein said treatment comprises oral administration of said composition (C) to the subject.

6. The composition (C) for use according to claim 5, wherein the subject is of an age comprised between 0 and 18 years, preferably between 1 and 14 years.

7. The composition (C) for use according to one of the preceding claim 5 or 6, wherein the composition further comprises bacterial strains belonging to the genus Oscillospira.

8. A pharmaceutical preparation or medical device or food supplement comprising a composition (C) which comprises (I) an effective amount of a mixture comprising, or consisting of, at least one bacterial strain selected from among the bacterial strains belonging to the species B. adolescentis selected from the group comprising, or consisting of:
- (i) Bifidobacterium adolescentis BA06, deposited with the Institute DSMZ in Germany by Probiotical S.p.A. on 7.4.2017 and having the deposit no. 32479; and
- (ii) Bifidobacterium adolescentis BA07, deposited with the Institute DSMZ in Germany by Probiotical S.p.A. on 21.4.2017 and having the deposit no. 32491; or
from among the bacterial strains belonging to the species B. bifidum selected from the group comprising, or consisting of:
- (iii) Bifidobacterium bifidum BB07, deposited with the Institute DSMZ in Germany by Probiotical S.p.A. on 7.4.2017 and having the deposit no. 32480; and
- (iv) Bifidobacterium bifidum BB08, deposited with the Institute DSMZ in Germany by Probiotical S.p.A. on 7.4.2017 and having the deposit no. 32481; or
from among the bacterial strains belonging to the species B. longum selected from the group comprising, or consisting of:
- (v) Bifidobacterium longum BL22, deposited with the Institute DSMZ in Germany by Probiotical S.p.A. on 7.4.2017 and having the deposit no. 32482; and
(vi) Bifidobacterium longum BL23, deposited with the Institute DSMZ in Germany by Probiotical S.p.A. on 7.4.2017 and having the deposit no. 32483; and
(II) at least one excipient suitable for pharmaceutical and/or food use.

9. The pharmaceutical preparation or medical device or food supplement according to claim 8, wherein the mixture (i) of the composition (C) comprises bacterial strains belonging to a species selected from the group consisting of or, alternatively, comprising each of the species Bifidobacterium longum, Bifidobacterium adolescentis and Bifidobacterium bifidum.

10. The pharmaceutical preparation or medical device or food supplement according to claim 8 or 9, wherein the mixture (i) of the composition (C) further comprises bacteria belonging to the genus Oscillospira.

## Patentansprüche

1. Bakterienstämme gehörend der Spezies *B*. *adolescentis*, die aus der Gruppe ausgewählt ist, die Folgendes umfasst oder daraus besteht:
- (i) *Bifidobacterium adolescentis* BA06, hinterlegt beim Institut DSMZ in Deutschland durch Probiotical S.p.A. am 7.4.2017 und mit der Hinterlegungsnummer 32479; und
- (ii) *Bifidobacterium adolescentis* BA07, hinterlegt beim Institut DSMZ in Deutschland durch Probiotical S.p.A. am 21.4.2017 und mit der Hinterlegungsnummer 32491.

2. Bakterienstämme gehörend der Spezies B. *bifidum,* die aus der Gruppe ausgewählt ist, die Folgendes umfasst oder daraus besteht:
- (iii) *Bifidobacterium bifidum* BB07, hinterlegt bei dem Institut DSMZ in Deutschland durch Probiotical S.p.A. am 7.4.2017 und mit der Hinterlegungsnummer 32480; und
- (iv) *Bifidobacterium bifidum* BB08, hinterlegt beim Institut DSMZ in Deutschland durch Probiotical S.p.A. am 7.4.2017 und mit der Hinterlegungsnummer 32481.

3. Bakterienstämme gehörend der Spezies B. *longum,* die aus der Gruppe ausgewählt ist, die Folgendes umfasst oder daraus besteht:
- (v) *Bifidobacterium longum* BL22, hinterlegt beim Institut DSMZ in Deutschland durch Probiotical S.p.A. am 7.4.2017 und mit der Hinterlegungsnummer 32482; und
- (vi) *Bifidobacterium longum* BL23, hinterlegt beim Institut DSMZ in Deutschland durch Probiotical S.p.A. am 7.4.2017 und mit der Hinterlegungsnummer 32483.

4. Bakterienstämme nach einem der Ansprüche 1-3, wobei die Bakterienstämme, die zu den Spezies *Bifidobacterium adolescentis*, *Bifidobacterium bifidum* und *Bifidobacterium longum* gehören, zur Verwendung bei der präventiven und/oder kurativen Behandlung von Symptomen und/oder Pathologien dienen, die von einem metabolischen Syndrom, einer vergrößerten Leber, einer nicht-alkoholischen Fettlebererkrankung (NAFLD) und/oder einer nicht-alkoholischen Steatohepatitis (NASH) in einem Subjekt abgeleitet oder damit verbunden sind; wobei die Behandlung vorzugsweise die orale Verabreichung der Stämme an das Subjekt umfasst.

5. Zusammensetzung (C), die eine wirksame Menge einer Mischung umfasst, die mindestens einen Bakterienstamm umfasst oder daraus besteht, der aus den Bakterienstämmen ausgewählt ist, die zu der Spezies *B*. *adolescentis* gehören, die aus der Gruppe ausgewählt ist, die Folgendes umfasst oder daraus besteht:
- (i) *Bifidobacterium adolescentis* BA06, hinterlegt beim Institut DSMZ in Deutschland durch Probiotical S.p.A. am 7.4.2017 und mit der Hinterlegungsnummer 32479; und
- (ii) *Bifidobacterium adolescentis* BA07, hinterlegt beim Institut DSMZ in Deutschland durch Probiotical S.p.A. am 21.4.2017 und mit der Hinterlegungsnummer 32491; oder
aus der Gruppe der Bakterienstämme, die zu der Spezies B. *bifidum* gehören, die aus der Gruppe ausgewählt ist, die Folgendes umfasst oder daraus besteht:
- (iii) *Bifidobacterium bifidum* BB07, hinterlegt bei dem Institut DSMZ in Deutschland durch Probiotical S.p.A. am 7.4.2017 und mit der Hinterlegungsnummer 32480; und
- (iv) *Bifidobacterium bifidum* BB08, hinterlegt beim Institut DSMZ in Deutschland durch Probiotical S.p.A. am 7.4.2017 und mit der Hinterlegungsnummer 32481; oder
aus der Gruppe der Bakterienstämme, die zu der Spezies B. *longum* gehören, die aus der Gruppe ausgewählt ist, die Folgendes umfasst oder daraus besteht:
- (v) *Bifidobacterium longum* BL22, hinterlegt beim Institut DSMZ in Deutschland durch Probiotical S.p.A. am 7.4.2017 und mit der Hinterlegungsnummer 32482; und
- (vi) *Bifidobacterium longum* BL23, hinterlegt beim Institut DSMZ in Deutschland durch Probiotical S.p.A. am 7.4.2017 und mit der Hinterlegungsnummer 32483;
zur Verwendung bei der präventiven und/oder kurativen Behandlung von Symptomen und/oder Pathologien, die von einem metabolischen Syndrom, einer vergrößerten Leber, einer nicht-alkoholischen Fettlebererkrankung (NAFLD) und/oder einer nicht-alkoholischen Steatohepatitis (NASH) in einem Subjekt abgeleitet oder damit verbunden sind, wobei die Behandlung die orale Verabreichung der Zusammensetzung (C) an das Subjekt umfasst.

6. Zusammensetzung (C) zur Verwendung nach Anspruch 5, wobei das Subjekt im Alter zwischen 0 und 18 Jahren, vorzugsweise zwischen 1 und 14 Jahren, ist.

7. Zusammensetzung (C) zur Verwendung nach einem der vorhergehenden Ansprüche 5 oder 6, wobei die Zusammensetzung ferner Bakterienstämme umfasst, die zur Gattung *Oscillospira* gehören.

8. Pharmazeutisches Präparat oder medizinische Vorrichtung oder Nahrungsergänzungsmittel, umfassend eine Zusammensetzung (C), die (I) eine wirksame Menge einer Mischung umfasst, die mindestens einen Bakterienstamm umfasst oder daraus besteht, der aus den Bakterienstämmen ausgewählt ist, die zu der Spezies *B*. *adolescentis* gehören, die aus der Gruppe ausgewählt ist, die Folgendes umfasst oder daraus besteht:
- (i) *Bifidobacterium adolescentis* BA06, hinterlegt beim Institut DSMZ in Deutschland durch Probiotical S.p.A. am 7.4.2017 und mit der Hinterlegungsnummer 32479; und
- (ii) *Bifidobacterium adolescentis* BA07, hinterlegt beim Institut DSMZ in Deutschland durch Probiotical S.p.A. am 21.4.2017 und mit der Hinterlegungsnummer 32491; oder
aus der Gruppe der Bakterienstämme, die zu der Spezies B. *bifidum* gehören, die aus der Gruppe ausgewählt ist, die Folgendes umfasst oder daraus besteht:
- (iii) *Bifidobacterium bifidum* BB07, hinterlegt bei dem Institut DSMZ in Deutschland durch Probiotical S.p.A. am 7.4.2017 und mit der Hinterlegungsnummer 32480; und
- (iv) *Bifidobacterium bifidum* BB08, hinterlegt beim Institut DSMZ in Deutschland durch Probiotical S.p.A. am 7.4.2017 und mit der Hinterlegungsnummer 32481; oder
aus der Gruppe der Bakterienstämme, die zu der Spezies B. *longum* gehören, die aus der Gruppe ausgewählt ist, die Folgendes umfasst oder daraus besteht:
- (v) *Bifidobacterium longum* BL22, hinterlegt beim Institut DSMZ in Deutschland durch Probiotical S.p.A. am 7.4.2017 und mit der Hinterlegungsnummer 32482; und
- (vi) *Bifidobacterium longum* BL23, hinterlegt beim Institut DSMZ in Deutschland durch Probiotical S.p.A. am 7.4.2017 und mit der Hinterlegungsnummer 32483; und
(II) mindestens einen Hilfsstoff, der für pharmazeutische und/oder Lebensmittelzwecke geeignet ist.

9. Pharmazeutisches Präparat oder medizinische Vorrichtung oder Nahrungsergänzungsmittel nach Anspruch 8, wobei die Mischung (i) der Zusammensetzung (C) Bakterienstämme umfasst, die zu einer Spezies gehören, die aus der Gruppe ausgewählt ist, die jeweils aus der Spezies *Bifidobacterium longum*, *Bifidobacterium adolescentis* und *Bifidobacterium bifidum* besteht oder diese alternativ umfasst.

10. Pharmazeutisches Präparat oder medizinische Vorrichtung oder Nahrungsergänzungsmittel nach Anspruch 8 oder 9, wobei die Mischung (i) der Zusammensetzung (C) ferner Bakterien umfasst, die zur Gattung *Oscillospira* gehören.

## Revendications

1. Souches bactériennes appartenant à l'espèce *B*. *adolescentis* choisies dans le groupe comprenant, ou constitué de :
- (i) *Bifidobacterium adolescentis* BA06, déposée auprès de l'Institut DSMZ en Allemagne par Probiotical S.p.A. le 7/04/2017 et ayant le numéro de dépôt 32479 ; et
- ii) *Bifidobacterium adolescentis* BA07, déposée auprès de l'Institut DSMZ en Allemagne par Probiotical S.p.A. le 21/04/2017 et ayant le numéro de dépôt 32491.

2. Souches bactériennes appartenant à l'espèce B. *bifidum* choisies dans le groupe comprenant, ou constitué de :
- (iii) *Bifidobacterium bifidum* BB07, déposée auprès de l'Institut DSMZ en Allemagne par Probiotical S.p.A. le 7/04/2017 et ayant le numéro de dépôt 32480 ; et
- (iv) *Bifidobacterium bifidum* BB08, déposée auprès de l'Institut DSMZ en Allemagne par Probiotical S.p.A. le 7/04/2017 et ayant le numéro de dépôt 32481.

3. Souches bactériennes appartenant à l'espèce B. *longum* choisies dans le groupe comprenant, ou constitué de :
- (v) *Bifidobacterium longum* BL22, déposée auprès de l'Institut DSMZ en Allemagne par Probiotical S.p.A. le 7/04/2017 et ayant le numéro de dépôt 32482 ; et
- (vi) *Bifidobacterium longum* BL23, déposée auprès de l'Institut DSMZ en Allemagne par Probiotical S.p.A. le 7/04/2017 et ayant le numéro de dépôt 32483.

4. Souches bactériennes selon l'une quelconque des revendications 1-3, dans lesquelles lesdites souches bactériennes appartenant aux espèces Bifidobacterium adolescentis, Bifidobacterium bifidum et *Bifidobacterium longum* sont à utiliser dans le traitement préventif et/ou curatif de symptômes et/ou de pathologies dérivant d', ou liés à, un syndrome métabolique, une hypertrophie du foie, une stéatose hépatique non alcoolique (NAFLD) et/ou une stéatohépatite non alcoolique (NASH) chez un sujet ; ledit traitement comprend de préférence une administration orale desdites souches au sujet.

5. Composition (C) qui comprend une quantité efficace d'un mélange comprenant, ou constitué d'au moins une souche bactérienne choisie parmi les souches bactériennes appartenant à l'espèce *B*. *adolescentis* choisies dans le groupe comprenant, ou constitué de :
- (i) *Bifidobacterium adolescentis* BA06, déposée auprès de l'Institut DSMZ en Allemagne par Probiotical S.p.A. le 7/04/2017 et ayant le numéro de dépôt 32479 ; et
- (ii) *Bifidobacterium adolescentis* BA07, déposée auprès de l'Institut DSMZ en Allemagne par Probiotical S.p.A. le 21/04/2017 et ayant le numéro de dépôt 32491 ; ou
parmi les souches bactériennes appartenant à l'espèce B. *bifidum* choisies dans le groupe comprenant, ou constitué de :
- (iii) *Bifidobacterium bifidum* BB07, déposée auprès de l'Institut DSMZ en Allemagne par Probiotical S.p.A. le 7/04/2017 et ayant le numéro de dépôt 32480 ; et
- (iv) *Bifidobacterium bifidum* BB08, déposé auprès de l'Institut DSMZ en Allemagne par Probiotical S.p.A. le 7/04/2017 et ayant le numéro de dépôt 32481 ; ou
parmi les souches bactériennes appartenant à l'espèce B. *longum* choisies dans le groupe comprenant, ou constitué de :
- (v) *Bifidobacterium longum* BL22, déposée auprès de l'Institut DSMZ en Allemagne par Probiotical S.p.A. le 7/04/2017 et ayant le numéro de dépôt 32482 ; et
- (vi) *Bifidobacterium longum* BL23, déposée auprès de l'Institut DSMZ en Allemagne par Probiotical S.p.A. le 7/04/2017 et ayant le numéro de dépôt 32483 ;
à utiliser dans le traitement préventif et/ou curatif de symptômes et/ou de pathologies dérivant de, ou liés à, un syndrome métabolique, une hypertrophie du foie, une stéatose hépatique non alcoolique (NAFLD) et/ou une stéatohépatite non alcoolique (NASH) chez un sujet, dans lequel ledit traitement comprend l'administration orale de ladite composition (C) au sujet.

6. Composition (C) à utiliser selon la revendication 5, dans laquelle le sujet est d'un âge compris entre 0 et 18 ans, de préférence entre 1 et 14 ans.

7. Composition (C) à utiliser selon l'une des revendications précédentes 5 ou 6, dans laquelle la composition comprend de plus des souches bactériennes appartenant au genre *Oscillospira.*

8. Préparation pharmaceutique ou dispositif médical ou complément alimentaire comprenant une composition (C) qui comprend (I) une quantité efficace d'un mélange comprenant, ou constitué d'au moins une souche bactérienne choisie parmi les souches bactériennes appartenant à l'espèce B. *adolescentis* choisies dans le groupe comprenant, ou constitué de :
- (i) *Bifidobacterium adolescentis* BA06, déposée auprès de l'Institut DSMZ en Allemagne par Probiotical S.p.A. le 7/04/2017 et ayant le numéro de dépôt 32479 ; et
- (ii) *Bifidobacterium adolescentis* BA07, déposée auprès de l'Institut DSMZ en Allemagne par Probiotical S.p.A. le 21/04/2017 et ayant le numéro de dépôt 32491 ; ou
parmi les souches bactériennes appartenant à l'espèce B. *bifidum* choisies dans le groupe comprenant, ou constitué de :
- (iii) *Bifidobacterium bifidum* BB07, déposée auprès de l'Institut DSMZ en Allemagne par Probiotical S.p.A. le 7/04/2017 et ayant le numéro de dépôt 32480 ; et
- (iv) *Bifidobacterium bifidum* BB08, déposé auprès de l'Institut DSMZ en Allemagne par Probiotical S.p.A. le 7/04/2017 et ayant le numéro de dépôt 32481 ; ou
parmi les souches bactériennes appartenant à l'espèce B. *longum* choisies dans le groupe comprenant, ou constitué de :
- (v) *Bifidobacterium longum* BL22, déposée auprès de l'Institut DSMZ en Allemagne par Probiotical S.p.A. le 7/04/2017 et ayant le numéro de dépôt 32482 ; et
- (vi) *Bifidobacterium longum* BL23, déposée auprès de l'Institut DSMZ en Allemagne par Probiotical S.p.A. le 7/04/2017 et ayant le numéro de dépôt 32483 ; et
(II) au moins un excipient adapté à un usage pharmaceutique et/ou alimentaire.

9. Préparation pharmaceutique ou dispositif médical ou complément alimentaire selon la revendication 8, dans laquelle/lequel le mélange (i) de la composition (C) comprend des souches bactériennes appartenant à une espèce choisie dans le groupe constitué des ou, alternativement, comprenant chacune les espèces *Bifidobacterium longum*, *Bifidobacterium adolescentis* et *Bifidobacterium bifidum.*

10. Préparation pharmaceutique ou dispositif médical ou complément alimentaire selon la revendication 8 ou 9, dans laquelle/lequel le mélange (i) de la composition (C) comprend de plus des bactéries appartenant au genre *Oscillospira.*
